# EUROPÄISCHE PATENTSCHRIFT

(11) **EP 2 943 239 B1**
(45) Veröffentlichungstag und Bekanntmachung des Hinweises auf die Patenterteilung: **07.03.2018**
(21) Anmeldenummer: 13811829.4
(22) Anmeldetag: 10.12.2013
(51) Int. Cl.: A61M 5/34, A61M 39/10

(54) **BEFESTIGUNGSELEMENT UND SPRITZE**
FASTENING ELEMENT AND SYRINGE
ÉLÉMENT DE FIXATION ET SERINGUE

(30) Priorität: 11.01.2013 DE 102013200339
(43) Veröffentlichungstag der Anmeldung: 18.11.2015
(73) Patentinhaber: Vetter Pharma-Fertigung GmbH & Co. KG, 88212 Ravensburg (DE)
(72) Erfinder: ZENKER, Jochen, 88212 Ravensburg (DE); HUND, Petra, 88276 Berg (DE)
(74) Vertreter: Gleiss, Alf-Olav
(86) Internationale Anmeldenummer: PCT/EP2013/076017
(87) Internationale Veröffentlichungsnummer: WO 2014/108262

(56) Entgegenhaltungen:
- EP-A1- 0 248 979
- WO-A1-2012/116790
- DE-A1-102009 007 250
- US-A- 5 702 374

## Beschreibung

Die Erfindung betrifft ein Befestigungselement gemäß Oberbegriff des Anspruchs 1, außerdem eine Spritze oder Karpule gemäß Anspruch 10.

Befestigungselemente der hier angesprochenen Art sind bekannt (DE 10 2009 007 250 A1, WO 2012/116790 A1, US 5,702,374). Sie dienen der Fixierung eines Ansatzelements an dem Fortsatz einer Spritze oder Karpule oder dergleichen, wobei das Ansatzelement eine Kanüle, ein Verbindungselement für eine Injektionseinrichtung oder dergleichen darstellen kann. Befestigungselemente dieser Art weisen einen ringförmigen Grundkörper auf, der beispielsweise mit einem Innengewinde versehen ist, in das ein Außengewinde einer Kanüle, eines Verbindungselements oder dergleichen angreifen kann, um ein Ansatzelement dieser Art an einer Spritze oder Karpule zu fixieren. Der ringförmige Grundkörper ist mit einem Ringelement versehen, welches schwenkbeweglich am Grundkörper angebracht und so ausgelegt ist, dass es den Fortsatz der Spritze oder Karpule im montierten Zustand des Befestigungselements umgreift und für eine sichere Fixierung des Befestigungselements am Fortsatz sorgt. Es hat sich herausgestellt, dass die Haltekräfte des Befestigungselements in einigen Fällen nicht ausreichend sind, sodass das Ansatzelement sich bereits beim Transport der Spritze oder Karpule, insbesondere aber bei der Handhabung der Spritze oder Karpule ungewollt löst, sodass der Inhalt der Spritze oder Karpule verunreinigt werden kann, was einerseits zu einem Verlust sehr wertvoller Medikamente oder aber auch zu einer gesundheitlichen Schädigung eines Patienten führen kann.

Aufgabe der Erfindung ist es daher, ein Befestigungselement der hier angesprochenen Art zu schaffen, welches diesen Nachteil vermeidet.

Zur Lösung dieser Aufgabe wird ein Befestigungselement der oben genannten Art geschaffen, welches die in Anspruch 1 genannten Merkmale umfasst. Es dient der Fixierung eines Ansatzelements an dem Fortsatz einer Spritze oder Karpule und weist einen Grundkörper auf, an dem ein Ringelement schwenkbeweglich angelenkt ist. Das Ringelement weist einen ringförmigen ersten Wandabschnitt auf, der über einen ersten Lagerbereich am Grundkörper angelenkt ist, außerdem einen ringförmigen zweiten Wandabschnitt, der über einen Lagerbereich mit dem ersten Wandabschnitt verbunden ist, wobei die beiden Wandabschnitte untereinander vorzugsweise federnd miteinander verbunden sind und einen Freiraum einschließen. Das Ringelement zeichnet sich dadurch aus, dass der erste Wandabschnitt mit dem Grundkörper federnd verbunden ist. Dadurch, dass das Ringelement zwei Wandabschnitte aufweist, zeichnet es sich durch eine hohe Flexibilität aus, die sicherstellt, dass das Ringelement sicher an den Fortsatz einer Spritze oder Karpule fixiert ist, sodass deren Transport beziehungsweise Handhabung möglich ist, ohne dass die Gefahr besteht, dass sich das Befestigungselement versehentlich vom Fortsatz der Spitze oder Karpule löst.

Bei einem bevorzugten Ausführungsbeispiel des Befestigungselements ist vorgesehen, dass das Ringelement segmentiert ausgebildet ist, wobei der erste und/oder zweite Wandabschnitt ebenfalls segmentiert ausgebildet ist. Es ist also nicht erforderlich, dass das Ringelement als geschlossener Ring ausgebildet ist. Es reicht, wenn es mehrere Segmente aufweist, die in montiertem Zustand des Befestigungselements den Fortsatz umgreifen, wobei der erste und/oder zweite Wandabschnitt ebenfalls segmentiert ausgebildet ist.

Bei einem weiteren bevorzugten Ausführungsbeispiel des Befestigungselements ist vorgesehen, dass der erste und/oder zweite Wandabschnitt durch die federnde Verbindung im unbelasteten Zustand in abgespeister Position gehalten wird. Das heißt, nachdem aufsetzen des Befestigungselements auf den Fortsatz einer Spritze oder Karpule wird durch die federnde Verbindung des ersten und/oder zweiten Wandabschnitts mit dem Grundkörper des Befestigungselements sichergestellt, dass das Ringelement sich an dem Fortsatz der Spritze oder Karpule so anlegt, dass eine sichere Fixierung dadurch gewährleistet ist, dass der Ringbereich so an den Fortsatz angelegt wird, dass er sicheren Halt an dem mindestens einen Vorsprung an der Außenfläche des Fortsatzes findet.

Ein besonders bevorzugtes Ausführungsbeispiel des Befestigungselements zeichnet sich dadurch aus, dass zumindest der zweite Wandabschnitt, der an dem Fortsatz in montiertem Zustand des Befestigungselements anliegt, mittels eines 2-Komponten-Spritzgussverfahrens herstellbar ist, wobei härtere und weichere Kunststoffkomponenten verwendet werden. Durch die härteren Kunststoffkomponenten wird sichergestellt, dass sich das Ringelement an der Außenfläche des Fortsatzes anlegt und sicheren Halt findet. Werden Kräfte auf das Befestigungselement ausgeübt, die ein Lösen desselben vom Fortsatz der Spritze oder Karpule bewirken könnten, verhakt sich das Ringelement sicher an dem mindestens einen Vorsprung auf der Außenfläche des Fortsatzes, weil die härteren Kunststoffkomponenten eine hohe Stabilität des Ringelements bewirken. Die weicheren Kunststoffkomponenten gewährleisten hohe Reibungskräfte an der Außenfläche des Fortsatzes, sodass eine sichere Fixierung des Befestigungselements gegeben ist.

Aufgabe der Erfindung ist außerdem, eine Spritze oder Karpule zu schaffen mittels derer sich die oben genannten Nachteile vermeiden lassen.

Zur Lösung der genannten Aufgabe wird eine Spritze oder Karpule vorgeschlagen, die einen Fortsatz mit mindestens einem Vorsprung auf der Außenfläche des Fortsatzes aufweist, sodass ein Befestigungselement sicher an dem Fortsatz fixiert werden kann.

Die Erfindung wird im Folgenden anhand der Zeichnung näher erläutert. Es zeigen:
- Figur 1: in perspektivischer Darstellung einen Längsschnitt durch ein erstes Ausführungsbeispiel eines Befestigungselements;
- Figur 2: in perspektivischer Darstellung einen Längsschnitt durch ein zweites Ausführungsbeispiel eines Befestigungselements und
- Figur 3: eine Unteransicht des zweiten Ausführungsbeispiels des Befestigungselements gemäß Figur 2.

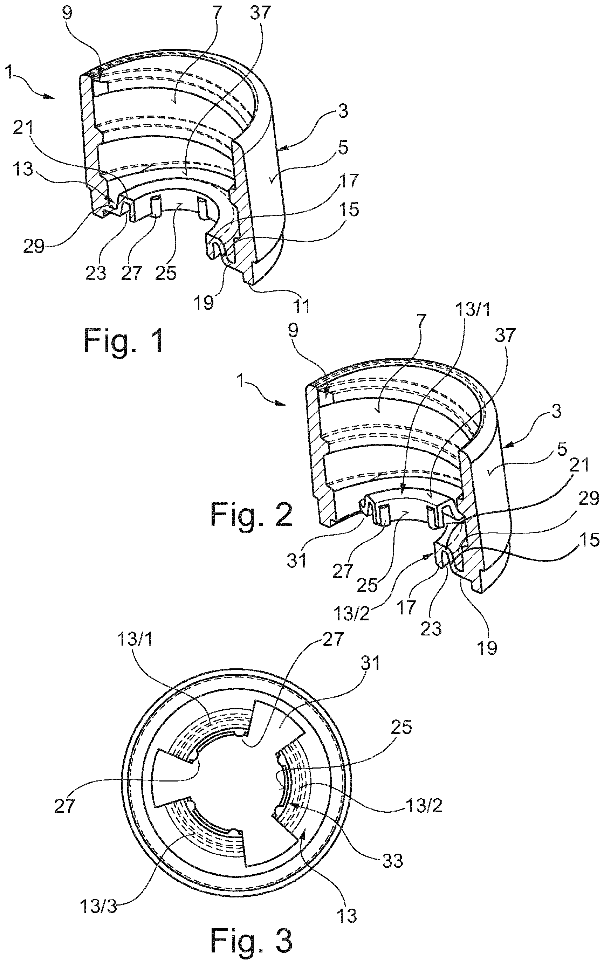

Aus Figur 1 ist ein erstes Ausführungsbeispiel eines Befestigungselements 1 ersichtlich, welches einen hier im Längsschnitt dargestellten ringförmigen Grundkörper 3 aufweist. Dieser zeigt eine Außenfläche 5 und eine Innenfläche 7, wobei bei dem hier dargestellten Ausführungsbeispiel auf der Innenfläche 7 eine Befestigungseinrichtung vorgesehen sein kann, die dazu dient, ein Ansatzelement sicher aufzunehmen, vorzugsweise eine Kanüle, oder aber auch ein Verbindungselement für eine Injektionseinrichtung oder dergleichen. Üblicherweise weist ein Befestigungselement 1 der hier angesprochenen Art auf seiner Innenfläche 7 ein Innengewinde 9 auf, welches mit einem Außengewinde eines Ansatzelements eines Kanüle zusammenwirkt, um die Kanüle einerseits sicher im Befestigungselement zu fixieren, während andererseits das Befestigungselement selbst an einem hier nicht dargestellten Fortsatz einer Spritze oder Karpule angebracht ist. Die Fixierung eines Ansatzelements im Befestigungselement kann auch mittels einer Bajonett-Verriegelung erfolgen

Zur Fixierung des Befestigungselements 1 an dem Fortsatz einer Spritze oder Karpule ist im Bereich des unteren freien Endes 11 des Grundkörpers 3 ein Ringelement 13 vorgesehen, welches einen ersten Wandabschnitt 15 und einen zweiten Wandabschnitt 17 umfasst. Das Ringelement 13 ist mit seinem ersten Wandabschnitt 15 an den Grundkörper 3 angelenkt und zwar über einen ersten Lagerbereich 19. Die beiden Wandabschnitte 15 und 17 sind miteinander verbunden und zwar über einen zweiten Lagerbereich 21.

Die beiden Wandabschnitte 15 und 17 sind über den zweiten Lagerbereich 21 so miteinander verbunden, dass zwischen den Wandabschnitten ein Freiraum 23 verbleibt, und das Ringelement 13, im Schnitt gesehen, V- beziehungsweise U-förmig ausgebildet ist. Der Freiraum 23 öffnet sich nach unten in Richtung auf das untere Ende 11 des Grundkörpers 3.

Der erste Lagerbereich 19 zwischen dem ersten Wandabschnitt 15 und dem Grundkörper 3 des Befestigungselements 1 ist federnd ausgebildet, sodass der erste Wandabschnitt 15 gegenüber der Innenfläche 7 des Grundkörpers 3 in abgespreizter Position gehalten wird und schräg nach innen ausgehend von der Innenfläche 7 verläuft.

Der zweite Lagerbereich 21 zwischen dem ersten Wandabschnitt 15 und dem zweiten Wandabschnitt 17 kann starr ausgebildet sein, sodass die beiden Wandabschnitte 15 und 17 auch beim Aufsetzen des Befestigungselements 1 auf einen Fortsatz einer hier nicht dargestellten Spritze oder Karpule U- beziehungsweise V-förmig zueinander angeordnet bleiben.

Vorzugsweise ist aber auch der zweite Lagerbereich 21 federnd ausgebildet, wobei die beiden Wandabschnitte 15 und 17 in unbelastetem Zustand, also während das Befestigungselement 1 nicht auf einen Fortsatz einer Spritze oder Karpule aufgesetzt ist, in abgespreiztem Zustand zueinander gehalten werden, sodass sich der in Figur 1 dargestellte Freiraum 23 zwischen den Wandabschnitten 15 und 17 ergibt. Die Elastizität im zweiten Lagerbereich 21 ist vorzugsweise jedoch so gewählt, dass beim Aufsetzen des Befestigungselements 1 auf einen Fortsatz einer Spritze oder Karpule die Wandabschnitte 15 und 17 gegen die Federelastizität im zweiten Lagerbereich 21 gegeneinander gerückt werden und dadurch quasi der Freiraum 23 geschlossen wird.

Beim Aufsetzen des Befestigungselements 1 auf einen Fortsatz wird außerdem aufgrund der Elastizität im ersten Lagerbereich 19 der erste Wandabschnitt 15 gegen die Innenfläche 7 des Grundkörpers 3 angelegt, sodass sich das Ringelement 13 quasi ziehharmonikaartig zusammenschiebt, während das Befestigungselement 1 auf einen Fortsatz einer Spritze oder Karpule aufgesetzt wird.

Vorzugsweise ist auf der Innenseite 25 des zweiten Wandabschnitts 17 mindestens ein Vorsprung 27 vorgesehen. Vorzugsweise sind über die Innenseite 25 in gleichmäßigem Abstand zueinander Vorsprünge 27 verteilt. Bei dem hier dargestellten Ausführungsbeispiel sind die Vorsprünge 27 streifenförmig ausgebildet und erstrecken sich über den wesentlichen Teil der Höhe des zweiten Wandabschnitts 17. Dabei ist vorzugsweise vorgesehen, dass die Vorsprünge 27 gewölbt, insbesondere kreisbogenförmig gewölbt ausgebildet sind, also eine teilzylindrische Fläche aufweisen, die sich über die Innenseite 25 des zweiten Wandabschnitts 17 erhebt.

Die Vorsprünge 27 können dadurch realisiert werden dass in den zweiten Wandabschnitt 17 Vertiefungen eingebracht und in die Elemente eingesetzt werden, welche die Vorsprünge 27 realisieren. Denkbar ist es auch, Elemente auf die Innenseite 25 des zweiten Wandabschnitts 17 Elemente aufzukleben, welche dann die Vorsprünge 27 verwirklichen.

Figur 1 zeigt, dass zwischen der Innenfläche 7 des Grundkörpers 3 und den vom ersten Lagerbereich 19 ausgehenden ersten Wandabschnitt 15 ein im Wesentlichen V-förmiger Spalt 29 vorhanden ist, der sich nach oben öffnet, also in eine Richtung, die vom unteren Ende 11 weggerichtet ist.

Es ist möglich, im ersten Lagerbereich 19, welcher den Boden des V-förmigen Spalts 29 bildet, Durchbrechungen vorzusehen, die hier allerdings nicht dargstellt sind. Diese dienen dazu, einen Ablauf für Flüssigkeiten zu gewährleisten, die bei Benutzung einer mit dem Befestigungselement 1 versehenen Spritze oder Karpule in den Spalt 29 gelangende Flüssigkeiten ablaufen zu lassen. Sie können in ihrer Form, insbesondere in ihrer - in Umfangsrichtung gesehenen - Ausdehnung variiert werden, um die Federeigenschaften des ersten Lagerbereichs 19 zu beeinflussen, also die Kräfte zu variieren, mit denen der erste Wandabschnitt 15 von der Innenfläche 7 abgespreizt wird.

Figur 2 zeigt ein abgewandeltes Ausführungsbeispiel des Befestigungselements 1. Gleiche Teile sind mit gleichen Bezugsziffern versehen, sodass insofern auf die Beschreibung zu Figur 1 verwiesen wird.

Der einzige Unterschied zwischen den beiden in den Figuren 1 und 2 dargestellten Ausführungsbeispielen des Befestigungselements besteht darin, dass das Ringelement 13 nicht als durchgehender Ring ausgebildet ist, wie dies anhand von Figur 1 erläutert wurde. Das Ringelement 13 ist vielmehr in einzelne, hier in drei Segmente unterteilt, von denen in Figur 2 die Segmente 13/1 und 13/2 zu erkennen sind. Zwischen jeweils zwei benachbarten Elmenten befindet sich ein Zwischenraum 31, dessen Breite ebenso wählbar ist, wie die Anzahl der Segmente des Ringelements. Es ist also sehr wohl möglich, mehr als drei in gleichmäßigem Umfangsabstand angeordnete Segmente von Ringelementen vorzusehen, oder aber auch nur zwei, die vorzugsweise gegenüberliegend im Bereich der Innenfläche 7 des Grundkörpers 3 angeordnet sind.

Figur 3 zeigt das zweite Ausführungsbeispiel des Befestigungselements 1 in Unteransicht. Es weist also ein Ringelement 13 mit drei Segmenten 13/1, 13/2 und 13/3 auf, die in gleichmäßigem Umfangsabstand zueinander angeordnet ist, wobei zwischen je zwei benachbarten Segmenten ein Zwischenraum 31 vorgesehen ist.

An den Figuren 2 und 3 ist ersichtlich, dass jedes der Ringsegmente 13/1, 13/2 und 13/3 zwei Vorsprünge 27 aufweist, die in den von den Ringsegmenten eingeschlossenen Freiraum im Befestigungselement 1 hineinragen, die also in den Innraum 32 des Befestigungselements 1 hineinragen.

Oben wurde ausgeführt, dass die Vorsprünge 27 in Ausnehmungen im zweiten Wandabschnitt 17 eingesetzte Elemente sein können, dass es aber auch möglich ist, auf die Innenseite 25 der Ringsegmente des Ringelements 13 Vorsprünge 27 aufzukleben.

Es ist auch möglich, ringsegmentförmige Vorsprungselemente 33 mit Vorsprüngen 27 in den zweiten Wandabschnitt 17 eines Ringsegments einzusetzen, sodass die Vorsprünge 27 in den vom Ringelement 13 beziehungsweise den Ringsegmenten 13/1 und 13/2 und 13/3 umschlossenen Innenraum 32 quasi radial hineinragen.

Es ist also möglich, Vorsprungselemente 33 mit Vorsprüngen 27 in die einzelnen Ringsegmente 13/1 bis 13/3 einzusetzen. Dies ist übrigens auch bei dem Ausführungsbeispiel nach Figur 1 möglich: Es kann ein ringförmiges Vorsprungselement in den zweiten Wandabschnitt 17 über den Freiraum 23 eingesetzt werden, sodass die Vorsprünge 27 nach innen ragen.

Besonders bevorzugt ist jedoch vorgesehen, dass zumindest der zweite Wandabschnitt 17 Kunststoff umfasst, vorzugsweise aus diesem besteht, und dass die Vorsprünge 27, gegebenenfalls auch das Vorsprungselement 33 ebenfalls Kunststoff umfasst, insbesondere aus diesem besteht.

Ganz besonders bevorzugt ist vorgesehen, dass der zweite Wandabschnitt 17 und die Vorsprünge 27, gegebenenfalls auch Vorsprungselemente 33 in einem 2-Kompontenen-Kunststoffspritzgussverfahren hergestellt werden.

Besonders kostengünstig ist es, das gesamte Befestigungselement 1 mit dem Ringelement 13, gegebenenfalls den Ringsegmenten 13/1 bis 13/3 mit den Vorsprüngen 27, die als Einzelelemente realisierbar sind oder von Vorsprungselementen 33 ausgehen, in einem 2-Komponenten-Spritzgussverfahren herzustellen.

Aus den Erläuterungen wird deutlich, dass auch der gesamte zweite Wandabschnitt 17 aus einem weicheren Material herstellbar ist, insbesondere dann, wenn der erste Wandabschnitt 15 vorzugsweise auch der zweite Lagerbereich 21 aus einem härteren Material hergestellt wird, sodass sich das Ringelement 13 beziehungsweise die Ringsegmente 13/1 bis 13/3 an einem Vorsprung abstützt beziehungsweise abstützen, wenn das Befestigungselement 1 auf einen Fortsatz einer Spritze oder Karpule fixiert ist und mit Zugkräften beaufschlagt wird, durch welche versucht wird, das Befestigungselement 1 von dem Fortsatz abzuziehen.

Zur Funktion des Befestigungselements 1, wie es anhand der Figuren 1 bis 3 erläutert wurde, ist Folgendes festzuhalten:
Die Figuren zeigen das Befestigungselement 1 in unbelastetem Zustand, also separat von einem Fortsatz einer Spritze oder Karpule. Diese weisen in der Regel einen Fortsatz auf, der zylindrisch, insbesondere aber leicht konisch ausgebildet ist, wobei der dickere Bereich des Fortsatzes in den Grundkörper der Spritze beziehungsweise Karpule übergeht.

In der Nähe dieses Übergangs ist vorzugsweise mindestens ein Vorsprung vorgesehen, der eine Schulter aufweist, die von dem freien Ende des Fortsatzes weggerichtet ist. Hinter diesem Fortsatz verrasstet das Befestigungselement 1 nach der Fixierung auf dem Fortsatz der Spritze oder Karpule.

Vorzugsweise ist in der Nähe des Übergangs zum Grundkörper der Spritze oder Karpule eine umlaufende Rinne in die Außenfläche des Fortsatzes eingebracht, die eine Bodenfläche aufweist, deren Außendurchmesser kleiner ist als der Außendurchmesser der Außenfläche des Fortsatzes im angrenzenden Bereich. Die Rinne ist damit im Wesentlichen U-förmig ausgebildet. Es reicht, wenn die Rinne lediglich eine seitliche Begrenzungswand aufweist, die auf der Seite der Rinne angeordnet ist, die zum freien Ende des Fortsatzes gerichtet ist. Die Rinne weist damit eine umlaufende Schulter auf, die als Vorsprung dient, an dem das Befestigungselement verrastend eingreift, wenn es auf den Fortsatz einer Spritze oder Karpule aufgeschoben wird.

Im Einzelnen ist dazu Folgendes festzuhalten:
Es wird im Folgenden davon ausgegangen, dass der Fortsatz einer Spritze oder Karpule eine umlaufende Rinne aufweist, deren in Richtung zum freien Ende des Fortsatz weisende Begrenzungsschulter einen Vorsprung bildet.

Der Innendurchmesser des Befestigungselements 1 ist so gewählt, dass der Grundkörper 3 auf den Fortsatz aufgeschoben werden kann. Dabei ist vorgesehen, dass die Innenseite 25 des zweiten Wandabschnitts 17 in entspanntem Zustand des Befestigungselements 1, wie er in den Figuren 1 bis 3 dargestellt ist, einen Innendurchmesser aufweist, der kleiner ist als der Außendurchmesser im Bereich der Rinne, die auf dem Fortsatz vorgesehen ist.

Wird also das Befestigungselement 1 mit seinem freien Ende 11 voran auf einen Fortsatz aufgeschoben, so drängt die Außenfläche des Fortsatzes den zweiten Wandabschnitt 17 radial nach außen, sodass das Ringelement 13 beziehungsweise seine Segmente 13/1 bis 13/3 gegen die Innenfläche 7 des Grundkörpers 3 verlagert werden. Dabei wird also der zweite Wandabschnitt 17 gegen den ersten Wandabschnitt 15 gedrängt, sodass sich bei elastischer Ausgestaltung des zweiten Lagerbereichs 21 die Weite des Freiraums 23 reduziert. Dabei können sich die beiden Wandabschnitte 17 und 15 aneinander legen. Dabei wird auch der erste Wandabschnitt 15 aufgeweitet, sodass die Weite des Spalts 29 reduziert wird, bis sich gegebenenfalls der erste Wandabschnitt 15 an die Innenfläche 7 des Grundkörpers 3 des Befestigungselements 1 anlegt. Das Ringelement 13 beziehungsweise dessen Segmente 13/1, 13/2 und 13/3 werden also quasi ziehharmonikaartig zusammengeschoben, während das Befestigungselement 1 auf den Fortsatz einer Spritze oder Karpule aufgesetzt wird.

Wird das Befestigungselement 1 in axialer Richtung auf dem Fortsatz der Spritze beziehungsweise Karpule verlagert in Richtung auf den Grundkörper der Spritze oder Karpule, so gelangt schließlich das Ringelement 13 in den Bereich des mindestens einen Vorsprungs beziehungsweise der Rinne, die auf der Außenfläche des Fortsatzes vorgesehen ist. Wird das Ringelement beziehungsweise dessen Segmente über den mindestens einen Vorsprung beziehungsweise die obere seitliche Begrenzungsfläche der Rinne hinweggeschoben, so kann das Ringelement 13 beziehungsweise dessen Segmente in Richtung auf die Bodenfläche der Rinne vorspringen. Mit anderen Worten: Der zweite Wandabschnitt 17 kann sich wieder zusammenziehen und - vom freien Ende des Fortsatzes aus gesehen - jenseits des mindestens einen Vorsprungs beziehungsweise jenseits der seitlichen Begrenzungskante der Rinne zurückfedern. Die Innenfläche 25 legt sich vorzugsweise an die Außenfläche des Fortsatzes jenseits des Vorsprungs beziehungsweise der seitlichen Begrenzungskante der Rinne an, sodass das Befestigungselement 1 sicher am Fortsatz der Spritze oder Karpule fixiert wird.

Die von dem unteren Ende 11 des Grundkörpers 3 des Befestigungselements 1 abgewandte Oberseite 37 des Ringelements 13 beziehungsweise der Ringsegmente 13/1 bis 13/3 liegt im montierten Zustand des Befestigungselements 1 an dem mindestens einen von der Außenfläche des Fortsatzes entsprechenden Vorsprung beziehungsweise an der oberen seitlichen Begrenzungsfläche der in die Außenfläche des Fortsatzes eingebrachten Rinne an, sodass ein axiales Abziehen des Befestigungselements 1 von dem Fortsatz praktisch ausgeschlossen ist. Wirken axiale Zugkräfte auf das Befestigungselement 1 so wird das Ringelement 13 beziehungsweise dessen Segmente 13/1 bis 13/3 durch den mindestens einen Vorsprung beziehungsweise die seitliche Begrenzungskante der Rinne festgehalten, sodass das Ringelement 13 beziehungsweise dessen Segmente 13/1 bis 13/3 unter Einwirkung axialer Kräfte nach innen in Richtung auf die Außenfläche des Fortsatzes um den ersten Lagerbereich 19 verkippt, sodass die Haltekräfte, mit denen das Befestigungselement 1 am Fortsatz fixiert wird, sich vergrößern.

Das Befestigungselement 1 ist vorzugsweise auch gegen ein unbeabsichtigtes Verdrehen auf dem Fortsatz einer Spritze beziehungsweise Karpule gesichert. Dies erfolgt bereits dadurch, dass das elastische Ringelement 13 beziehungsweise dessen Segmente beim Aufsetzen auf dem Fortsatz ausgedehnt werden und sich mit hoher Kraft aufgrund ihrer elastischen Rückstellkräfte an die Außenfläche des Fortsatzes anlegen. Insbesondere liegt natürlich der zweite Wandabschnitt 17 mit seiner Innenseite 25 an der Außenfläche des Fortsatzes an.

Zur Erhöhung der Reibungskräfte kann der zweite Wandabschnitt 17 zumindest auf seiner Innenseite 25, oder aber auch insgesamt aus einem weicheren Material bestehen, als es für den ersten Wandabschnitt 15 gewählt wird. Das weichere Material legt sich besser an die Außenfläche des Fortsatzes an und baut damit hohe Reibungskräfte auf. Das härtere Material des ersten Wandabschnitts 15 sorgt dafür, dass dieser - in axialer Richtung gesehen - stabil genug ist, axiale Zugkräfte abzufangen und ein Verkippen des Ringelements 13 beziehungsweise der Ringsegmente 13/1 bis 13/3 zu gewährleisten, damit das Befestigungselement 1 unter dem mindestens einen Vorsprung beziehungsweise der oberen seitlichen Begrenzungsfläche einer Rille in der Außenfläche des Fortsatzes gehalten wird.

Zur Erhöhung der Reibungskräfte zwischen dem zweiten Wandabschnitt 17 und der Außenfläche des Fortsatzes können einzelne Vorsprünge 27 auf der Innenseite 25 des zweiten Wandabschnitts 17 vorgesehen werden, die besonders bevorzugt aus einem weicheren Material bestehen und sich dadurch sehr gut an die Außenfläche des Fortsatzes anlegen und Reibungskräfte aufbauen, die ein unbeabsichtigtes Verdrehen des Befestigungselement 1 gegenüber dem Fortsatz der Spritze oder Karpule aber auch ein Abziehen in axialer Richtung verhindern.

Besonders hohe Widerstandskräfte werden aufgebaut, wenn der zweite Lagerbereich 21 zwischen dem ersten Wandabschnitt 15 und dem zweiten Wandabschnitt 17 relativ starr ausgelegt ist. Um die beim Aufsetzen des Befestigungselements 1 erforderlichen Kräfte zu reduzieren, ist dieser zweite Lagerbereich 21 jedoch vorzugsweise elastisch ausgebildet, sodass in unbelastetem Zustand die beiden Wandabschnitte 15 und 17 abgespreizt voneinander gehalten werden, und somit der Freiraum 23 gegeben ist.

Um die Haltekräfte zu variieren, kann die Elastizität des zweiten Lagerbereichs 21 aber auch des ersten Lagerbereichs 19 verändert werden. Gegebenenfalls können auch im ersten Lagerbereich 19 die oben angesprochenen Durchbrüche vorgesehen werden. Dadurch werden die Abspreizkräfte eingestellt, die versuchen, den ersten Wandabschnitt 15 in abgespreiztem Zustand von der Innenfläche 7 des Grundkörpers 3 zu halten beziehungsweise diesen gegen die Außenfläche eines Fortsatzes zu drängen.

Aus den Figuren 1 und 2 ist ersichtlich, dass der Grundkörper 3 im Bereich des freien Endes 11 verstärkt ausgebildet sein kann. Hier ist die Wand des Grundköpers 3 etwas dicker ausgelegt, als dies oberhalb des Ringelements 13 beziehungsweise der Ringsegmente 13/1 bis 13/3 der Fall ist.

## Patentansprüche

1. Befestigungselement zur Fixierung eines Ansatzelements an dem Fortsatz einer Spritze oder Karpule, der auf seiner Außenfläche mindestens einen Vorsprung aufweist, wobei das Befestigungselement (1) einen ringförmigen Grundkörper (3) aufweist, an dem ein Ringelement (13), so angelenkt ist, dass es den Fortsatz in montiertem Zustand umgreift, wobei das Ringelement (13)
- einen ringförmigen ersten Wandabschnitt (15) aufweist, der über einen ersten Lagerbereich (19) am Grundkörper (3) angelenkt ist, und
- einen ringförmigen zweiten Wandabschnitt (17) aufweist, der über einen zweiten Lagerbereich (21) an den ersten Wandabschnitt (15) angelenkt ist, wobei
- die beiden Wandabschnitte (15; 17) vorzugsweise federnd miteinander verbunden sind und einen Freiraum (23) zwischen sich einschließen, und wobei
- der erste Wandabschnitt (15) federnd mit dem Grundkörper (3) verbunden ist.

2. Befestigungselement nach Anspruch 1, **dadurch gekennzeichnet, dass** das Ringelement (13) segmentiert ausgebildet ist, wobei der erste und/oder zweite Wandabschnitt (15;17) ebenfalls segmentiert ausgebildet ist.

3. Befestigungselement nach Anspruch 1 oder 2, **dadurch gekennzeichnet, dass** der erste und/oder zweite Wandabschnitt (15;17) durch die federnde Verbindung in unbelasteten Zustand in abgespreizter Position gehalten wird.

4. Befestigungselement nach einem der vorhergehenden Ansprüche, **dadurch gekennzeichnet, dass** der erste und/oder zweite Lagerbereich (19,21) eine schwenkbewegliche Anlenkung des ersten beziehungsweise zweiten Wandabschnitts (15;17) ermöglicht.

5. Befestigungselement nach Anspruch 4, **dadurch gekennzeichnet, dass** die Schwenkbeweglichkeit in den Lagerbereichen (19,21) verschieden ist.

6. Befestigungselement nach einem der vorhergehenden Ansprüche, **dadurch gekennzeichnet, dass** zumindest der zweite Wandabschnitt (17) Kunststoff aufweist, vorzugsweise aus Kunststoff besteht.

7. Befestigungselement nach Anspruch 6, **dadurch gekennzeichnet, dass** zumindest der zweite Wandabschnitt (17) härtere und weichere Abschnitte aufweist.

8. Befestigungselement nach Anspruch 6 oder 7, **dadurch gekennzeichnet, dass** zumindest der zweite Wandabschnitt (17) mittels eines 2-Komponenten-Spritzgussverfahrens herstellbar ist, wobei vorzugsweise härtere und weichere Kunststoffkomponenten verwendet werden.

9. Befestigungselement nach einem der vorhergehenden Ansprüche, **dadurch gekennzeichnet, dass** zumindest im ersten Lagerbereich (19) wenigstens ein Durchbruch vorgesehen ist.

## Claims

1. Fastening element for fixing an attachment element on the extension piece of a syringe or carpule, said extension piece having, on its outer face, at least one projection, wherein the fastening element (1) has an annular main body (3) on which a ring element (13) is articulated such that it engages around the extension piece in the mounted state, wherein the ring element (13)
- has an annular first wall portion (15), which is articulated on the main body (3) via a first bearing area (19), and
- has an annular second wall portion (17), which is articulated on the first wall portion (15) via a second bearing area (21), wherein
- the two wall areas (15; 17) are preferably connected to each other resiliently and enclose between them a free space (23), and wherein
- the first wall area (15) is connected resiliently to the main body (3).

2. Fastening element according to claim 1, **characterized in that** the ring element (13) has a segmented design, wherein the first and/or second wall area (15; 17) likewise has a segmented design.

3. Fastening element according to claim 1 or 2, **characterized in that** the first and/or second wall area (15; 17) is held in a spread-open position, by the resilient connection, in an unloaded state.

4. Fastening element according to one of the preceding claims, **characterized in that** the first and/or second bearing area (19, 21) permits a pivotable articulation of the first and/or second wall area (15; 17).

5. Fastening element according to claim 4, **characterized in that** the pivotability in the bearing areas (19, 21) is different.

6. Fastening element according to one of the preceding claims, **characterized in that** at least the second wall area (17) comprises plastic, preferably consists of plastic.

7. Fastening element according to claim 6, **characterized in that** at least the second wall area (17) has harder and softer portions.

8. Fastening element according to claim 6 or 7, **characterized in that** at least the second wall area (17) can be produced by means of a two-component injection molding technique, wherein components made of harder and softer plastic are preferably used.

9. Fastening element according to one of the preceding claims, **characterized in that** at least one aperture is provided at least in the first bearing area (19).

## Revendications

1. Élément de fixation pour fixer un élément de raccordement au prolongement d'une seringue ou une carpule présentant au moins une saillie sur sa surface extérieure, l'élément de fixation (1) ayant un corps de base (3) annulaire sur lequel un élément annulaire (13) est articulé de telle manière qu'il vient en prise autour du prolongement dans l'état installé, dans lequel l'élément annulaire (13)
- présente une première partie de paroi (15) annulaire, articulée au corps de base (3) au moyen d'une première région de palier (19), et
- une seconde partie de paroi (17) annulaire, articulée à la première partie de paroi (15) au moyen d'une seconde région de palier (21),
- les deux parties de parois (15 ; 17) étant reliées l'une avec l'autre, de préférence de manière élastique, et entourant un espace libre (23) entre elles, et
- la première partie de paroi (15) étant reliée de manière élastique au corps de base (3).

2. Élément de fixation selon la revendication 1, **caractérisé en ce que** l'élément annulaire (13) est configuré de manière segmentée, la première et/ou la seconde partie de paroi (15 ; 17) aussi étant configurées de manière segmentée.

3. Élément de fixation selon la revendication 1 ou 2, **caractérisé en ce que** la première et/ou la seconde partie de paroi (15 ; 17) sont tenues dans la position écartée par la liaison élastique dans l'état sans charge.

4. Élément de fixation selon l'une quelconque des revendications précédentes, **caractérisé en ce que** la première et/ou seconde région de palier (19, 21) permet(tent) l'articulation pivotable de la première et la seconde partie de paroi (15 ; 17) respectivement.

5. Élément de fixation selon la revendication 4, **caractérisé en ce que** la mobilité de pivotement est différente dans les régions de palier (19, 21).

6. Élément de fixation selon l'une quelconque des revendications précédentes, **caractérisé en ce qu'**au moins la seconde partie de paroi (17) comporte de la matière plastique, et de préférence consiste de la matière plastique.

7. Élément de fixation selon la revendication 6, **caractérisé en ce qu'**au moins la seconde partie de paroi (17) présente des parties plus dures et plus souples.

8. Élément de fixation selon la revendication 6 ou 7, **caractérisé en ce qu'**au moins la seconde partie de paroi (17) peut être fabriquée au moyen d'un procédé de moulage par injection à deux composants, en utilisant de préférence des composants en plastique plus durs et plus souples.

9. Élément de fixation selon l'une quelconque des revendications précédentes, **caractérisé en ce qu'**au moins une percée est prévue au moins dans la première région de palier (19).
